(19) Europäisches Patentamt
European Patent Office
Office européen des brevets

(11) Publication number: **0 264 901 B1**

(12) # EUROPEAN PATENT SPECIFICATION

(45) Date of publication of patent specification: **10.08.94**

(51) Int. Cl.[5]: **C07D 233/52**, C07D 233/28, A61K 31/415

(21) Application number: **87115324.3**

(22) Date of filing: **20.10.87**

(54) **Acylation products of bis(2-imidazolin-2-ylhydrazones) of 9,10-anthracenedicarboxaldehyde.**

(30) Priority: **23.10.86 US 922220**

(43) Date of publication of application:
**27.04.88 Bulletin 88/17**

(45) Publication of the grant of the patent:
**10.08.94 Bulletin 94/32**

(84) Designated Contracting States:
**AT BE CH DE ES FR GB GR IT LI NL SE**

(56) References cited:
**EP-A- 0 018 850**
**EP-A- 0 073 960**
**DE-A- 2 850 822**
**US-A- 3 996 372**

**CHEMICAL ABSTRACTS, vol. 96, no. 19, 10th may 1982 page 746, abstract no. 162593e, Columbus, Ohio, U:S; K.C. MURDOCK et al.: "Antitumor agents. II. Bis (guanylhydrazones) of anthracene-9,10-dicarboxaldehydes"; & J. MED. CHEM. 1982, 25(5), 505-518**

(73) Proprietor: **AMERICAN CYANAMID COMPANY**
**One Cyanamid Plaza**
**Wayne, NJ 07470-8426(US)**

(72) Inventor: **Murdock, Keith Chadwick**
**15 Birch Street**
**Pearl River New York 10965(US)**

(74) Representative: **Wächtershäuser, Günter, Prof. Dr.**
**Patentanwalt**
**Tal 29**
**D-80331 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention is concerned with new organic compounds which are N-acylated derivatives of bis(2-imidazolin-2-ylhydrazones) of 9,10-anthracenedicarboxaldehyde.

The unacylated precursor compounds are disclosed in U.S. Patent 4,258,181 and DE-A-2850822. Bis-(guanylhydrazones) of anthracen-9,10-dicarboxaldehydes are useful as antitumor agents (see J. Med. Chem. 1982, 25, 505 - 518). However, the known compounds frequently show side effects, e.g. a painful phlebitis near the site of injection.

This invention is concerned with compounds of the formula:

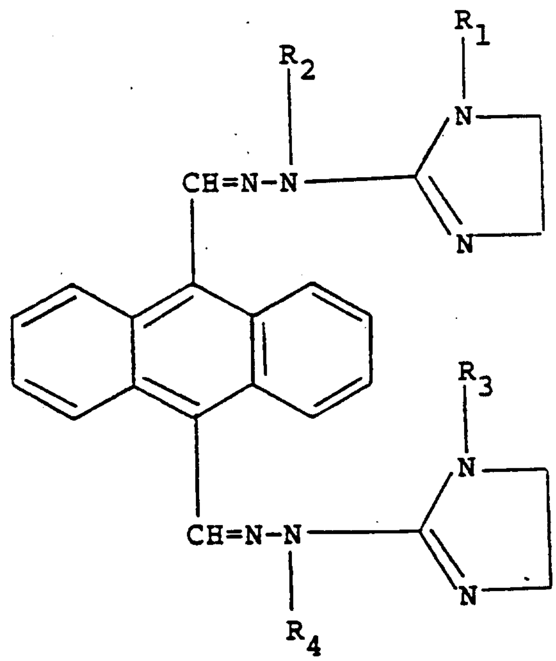

wherein $R_1$ and $R_3$ are the same or different and are: hydrogen, alkyl($C_1$-$C_6$),

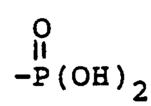, 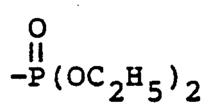, 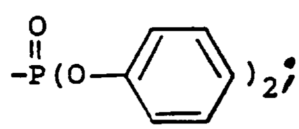;

and wherein only one of $R_1$ and $R_3$ may be hydrogen or alkyl($C_1$-$C_6$); $R_2$ and $R_4$ are the same or different and are: hydrogen, alkyl($C_1$-$C_4$) or

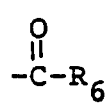

((where $R_6$ is hydrogen, alkyl($C_1$-$C_6$), phenyl, mono-substituted phenyl (wherein the substituent may be in the ortho, meta or para position and is fluoro, nitro, alkyl ($C_1$-$C_6$), alkoxy($C_1$-$C_3$) or cyano), pentafluorophenyl, naphthyl, furanyl or -$CH_2OCH_3$)); together with the pharmacologically acceptable salts thereof.

Special mention is made of the monophosphoramidic acids embraced by the foregoing formula. Their anti-tumor activity is coupled with a lack of painful phlebitis near the site of injection when administered to some warm-blooded animals.

The compounds of the present invention are obtainable as yellow to orange crystalline materials having characteristic melting points and absorption spectra, and which may be purified by recrystallization from

common organic solvents such as lower alkanols, dimethylformamide or methyl isobutyl ketone.

The compounds of the present invention may be readily prepared in accordance with the following reaction scheme:

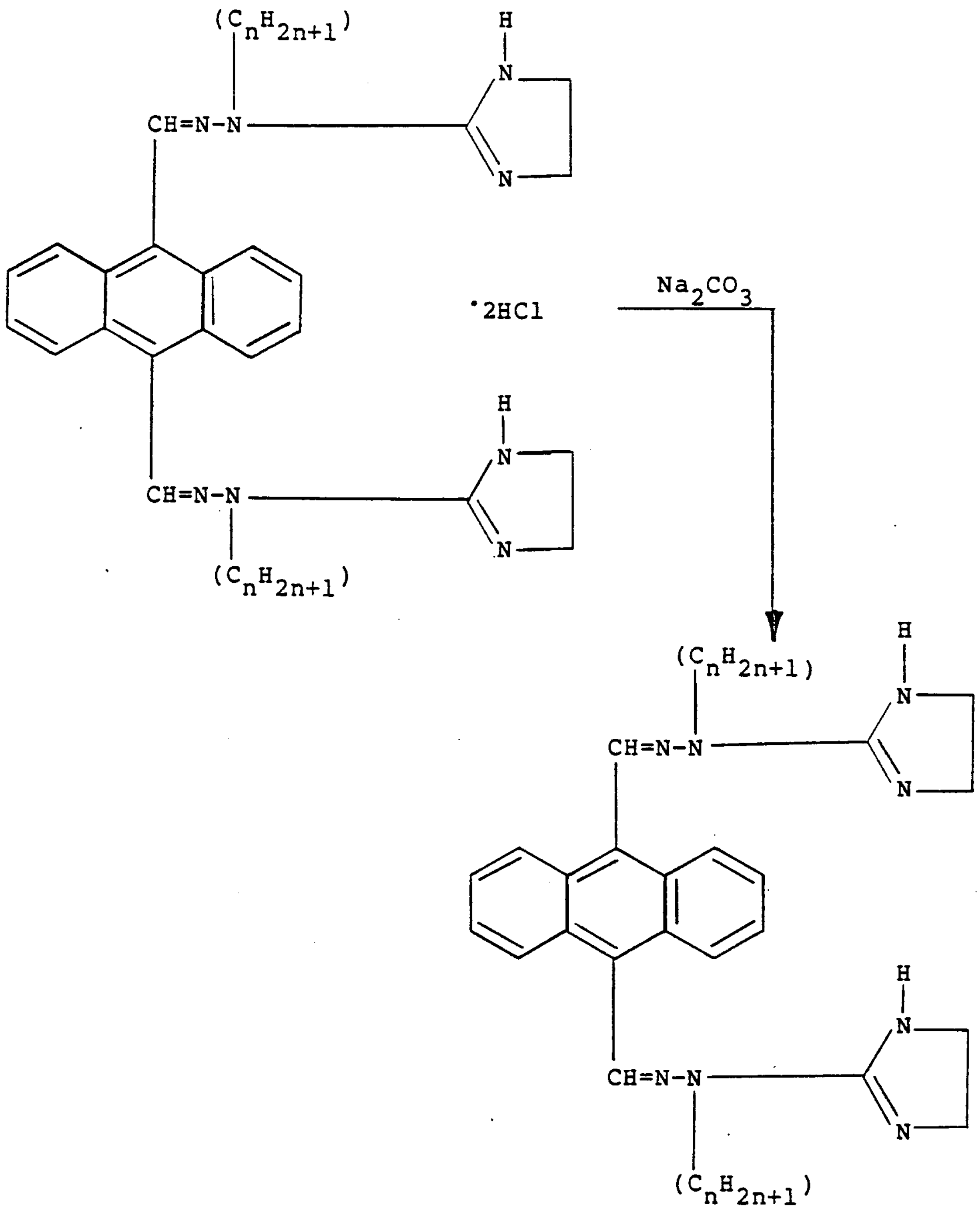


(3)

+

A (in excess)

(2)

(1)

Scheme (continued)

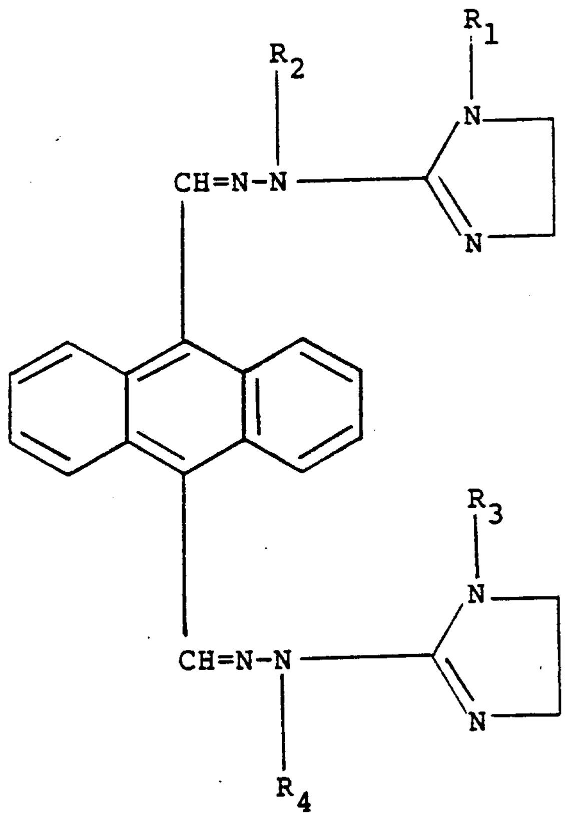

(1)

where A is an acylating agent including the diester of a phosphoric acid chloride and where $R_1$, $R_2$, $R_3$ and $R_4$ are as hereinbefore defined.

An acylating anhydride may be employed without the need of an acid-binding agent. However, when acid chlorides are used in the acylation process, a non-basic acid-binding agent is employed to prevent major formation of bis(2-imidazolin-2-ylhydrazone) of 9,10-anthracenedicarboxaldehyde dihydrochloride as an undesired byproduct. A convenient non-basic acid-binding agent which is used for this purpose is N,O-bis(trimethylsilyl) acetamide.

In accordance with the above reaction scheme, bis(2-imidazolin-2-ylhydrazone) of 9,10-anthracenedicarboxaldehyde dihydrochloride or an N,N'-dialkyl derivative (4), disclosed in U.S. Patent 4,258,181, is treated with an aqueous solution of sodium carbonate and is allowed to stand for several hours to precipitate the free base compound (3). The product is collected by filtration, then dried in vacuo at about 110 °C for about 15 hours.

When the acylating agent A is an anhydride the following procedure applies: The dried free base (3) is suspended and stirred in a dried solvent such as dichloromethane or N,N-dimethylformamide in an inert atmosphere, e.g., under nitrogen or argon then an excess of the anhydride (2) is added and stirring is continued until the solid is dissolved. The solution is allowed to stand at about 23 °C for 8-48 hours. The product (1) precipitates either spontaneously or after addition of ether or water, then is collected by filtration.

When the acylating agent (2) is an acid chloride such as diethyl chlorophosphate, the following procedure is used: The dried bis(2-imidazolin-2-ylhydrazone) of 9,10-anthracenedicarboxaldeyde free base (3) is suspended and stirred in dichloromethane under argon or nitrogen in a closed round bottom flask equipped with a stirrer and a rubber septum cap, then an acid binding agent such as N,O-bis(trimethylsilyl)-acetamide is added with stirring, using a weighed hypodermic syringe to inject the reagent through the rubber septum. Then the desired acid chloride is added in the same manner. The reaction mixture is stirred from three to sixty-four hours. The solution or suspension is chromatographed by dry column chromatography on alumina, and eluted with solvents such as dichloromethane, ethyl acetate, chloroform, acetone. The cuts are collected and evaluated by thin layer chromatography on silica gel using solvent systems such as 3/1, 19/1 or 39/1 of chloroform/methanol, then the cuts containing the desired products are evaporated and purified by conventional means.

When, for example, the alkyl esters of mono- and di-phosphinic acid derivatives are to be converted to the corresponding free phosphonic acids, in an elegant modification according to this invention, a triarylphosphine, preferably triphenylphosphine will be used with the cleaving agent, e.g. iodotrialkylsilane, to remove byproduct alkyl iodide, thus precluding alkylation in other positions. This will be exemplified hereinafter.

Certain in vivo testing systems and protocols have been developed by the National Cancer Institute for testing compounds to determine their suitability as antineoplastic agents. These have been reported in "Cancer Chemotherapy Reports", Part III, Vol. 3, No. 2 (1972), by Deran, Greenberg, MacDonald, Schumacher and Abbott. These protocols have established standardized screening tests which are generally followed in the field of testing for antitumor agents. Three of these systems are particularly significant to the present invention. They are lymphocytic leukemia P388, melanotic melanoma B16 and lymphocytic leukemia L1210. All of these neoplasms grow in mice. Generally, good antitumor activity, shown in these protocols by a percentage increase of mean survival times of the treated (T) animals over the control (C) animals, is predictive of similar results in human leukemias.

Lymphocytic leukemia P388 test

The animals used were BDFI mice all of one sex per test, weighing a minimum of 17 g and all within a 3 g weight range per test. There were 6 animals per test group. The tumor transplant was by intraperitoneal injection of 0.5 ml of diluted ascitic fluid containing $10^6$ cells of lymphocytic leukemia P388. The test compounds were administered intraperitoneally on days 1, 5 and 9 (relative to tumor inoculation) at various doses. The animals were weighed and survivors recorded on a regular basis for 60 days. The median survival time and the ratios for treated (T)/control (C) animals were calculated. The positive control compound was 5-fluorouracil, given as a 60 mg/kg injection hereinafter called positive control A. The results of this test with representative compounds of the present invention appear in Table I.

TABLE I

Lymphocytic Leukemia P388 Test

| Compound | Dose (mg/kg) | Median Survival (Days) | T/C x 100 % |
|---|---|---|---|
| [9,10-Anthracenediylbis [methylidyne-1-hydrazinyl-2-ylidene(4,5-dihydro-1H-imidazol-2,1-diyl)]]bisphosphonic acid, tetraphenyl ester | 200<br>100<br>50 | 16<br>15.5<br>13 | 160<br>155<br>130 |
| Control | - | 10 | - |
| Positive Control A | 60 | 17.5 | 175 |
| [9,10-Anthracenediylbis [methylidyne-1-hydrazinyl-2-ylidene(4,5-dihydro-1H-imidazole-2,1-diyl)]]bisphosphonic acid, tetraethyl ester | 100 | 12 | 120 |
| Control | - | 10 | 0 |
| Positive Control A | 60 | 17.5 | 175 |
| [9,10-Anthracenediylbis [methylidyne-1-hydrazinyl-2-ylidene(4,5-dihydro-1H-imidazole-2,1-diyl)]]bisphosphonic acid | 25<br>12.5<br>6.25<br>3.12<br>1.56<br>0.78 | 36.5<br>27.5<br>22<br>18<br>17<br>17.5 | 365<br>275<br>220<br>180<br>170<br>175 |
| Control | - | 10 | - |
| Positive Control A | 60 | 17.5 | 175 |

TABLE I (continued)

| Compound | Dose (mg/Kg) | Median Survival (Days) | T/C x 100 % |
|---|---|---|---|
| [2-[[[10-[[4,5-dihydro-1H-imidazol-2-ylethyl-hydrazono]methyl]-9-anthracenyl]methylene]hydrazino]-4,5-dihydro--1H-imidazol-1-yl]phosphonic acid, hydroiodide | 50 | 25.5 | 232 |
| | 25 | 25 | 227 |
| | 12.5 | 25 | 227 |
| | 6.3 | 24 | 218 |
| | 3.2 | 22.5 | 205 |
| | 1.6 | 18 | 164 |
| | 0.8 | 21.5 | 195 |
| | 0.4 | 18 | 164 |
| Control | - | 11 | - |
| Positive Control A | 60 | 22 | 200 |
| [2[[[10-[[4,5-dihydro-1H-imidazol-2-yl)hydrazono]methyl]9-anthracenyl]methylene]hydrazino]-4,5-dihydro-1H-imidazol-1-yl]phosphonic acid, hydroiodide | 25 | >60 | >545 |
| | 12.5 | >47 | >427 |
| | 6.3 | 22 | 200 |
| | 3.2 | 24 | 218 |
| | 1.6 | 24.5 | 223 |
| | 0.8 | 19 | 173 |
| | 0.4 | 21 | 191 |
| Control | - | 11 | - |
| Positive Control A | 60 | 22 | 200 |

TABLE I (continued)

| Compound | Dose (mg/Kg) | Median Survival (Days) | T/C x 100 % |
|---|---|---|---|
| [2-[[[10-[[4,5-dihydro-1H-imidazol-2-yl)hydrazono]methyl]-9-anthracenyl]methylene]hydrazino]-4,5-dihydro-1H-imidazol-1-yl]phosphonic acid, diethyl ester | 100 | >34 | >309 |
| | 50 | >38 | >345 |
| | 25 | >29 | >264 |
| | 12.5 | 19 | 173 |
| | 6.3 | 21.5 | 195 |
| | 3.2 | 23.5 | 214 |
| | 1.6 | 22.5 | 205 |
| | 0.8 | 19 | 173 |
| | 0.4 | 18 | 164 |
| Control | - | 11 | - |
| Positive Control A | 60 | 22 | 200 |

Melanotic Melanoma Bl6

The animals used were BDFl mice, all of one sex, weighing a minimum of 17 g, and all within a 3 g weight range. There were normally 12 animals per test group, and 18 animals per control group. A one gram portion of melanotic melanoma Bl6 tumor was homogenized in 10 ml of Eagle's Minimum Essential Medium, supplemented with 2% fetal calf serum, and a 0.5 ml aliquot of the homogenate was implanted intraperitoneally into each test mouse. The test compounds were administered intraperitoneally on days 1, 5 and 9 (relative to tumor inoculation) at various doses. The animals were weighed and survivors recorded on a regular basis for 60 days. The median survival time and the ratio of survival time for treated (T)/control (C) animals were calculated. The positive control compound was bis(2-imidazolin-2-ylhydrazone) of 9,10-anthracenedicarboxaldehyde dihydrochloride (hereinafter called positive control B) given as intraperitoneal injection at a dose of 25 mg/kg on days 1, 5 and 9 (relative to tumor inoculation). The results of this test with a representative compound of the present invention appear in Table II.

TABLE II

| Melanotic Melanoma Bl6 Test | | | |
|---|---|---|---|
| Compound | Dose (mg/kg) | Median Survival (Days) | T/C x 100 % |
| [9,10-Anthracenediylbis[methylidyne-1-hydrazinyl-2-ylidene(4,5-dihydro--1H-imidazole-2,1-diyl)]]bisphosphonic acid | 25 | 37.5 | 163 |
| | 6.25 | 35.5 | 154 |
| | 1.56 | 29 | 126 |
| Control | - | 23 | - |
| Positive Control B | 25 | 53 | 230 |

Lymphocytic Leukemia L1210 Test

The animals used were BDFl mice, all of one sex, weighing a minimum of 17 g and all within a 3 g weight range. There were 6 mice per test group and 18 in control groups. The tumor transplant was by intraperitoneal injection of 0.5 ml of diluted ascites containing $10^5$ viable L1210 leukemia cells per mouse. The test compounds were administered intraperitoneally on days 1, 5 and 9 (relative to tumor inoculation) at various doses. The animals were weighed and survivors recorded on a regular basis for 30 days. The mean survival time and the ratio of survival time for treated (T)/control(C) animals were calculated. The positive control compound A was 5-fluorouracil given intraperitoneally at 60 mg/kg. The results of this test appear in Table III.

TABLE III

| Lymphocytic Leukemia L1210 Test | | | |
|---|---|---|---|
| Compound | Dose (mg/kg) | Median Survival (Days) | T/C x 100 % |
| [9,10-Anthracenediylbis[methylidyne-1-hydrazinyl-2-ylidene(4,5-dihydro--1H-imidazole-2,1-diyl)]]bisphosphonic acid | 25 | 17 | 200 |
| | 12.5 | 13.3 | 156 |
| | 6.25 | 13.2 | 155 |
| | 3.12 | 12.2 | 144 |
| | 1.56 | 11.5 | 135 |
| | 0.78 | 10.7 | 126 |
| Control | - | 8.5 | - |
| Positive Control A | 60 | 16.8 | 198 |

Also embraced within the purview of the present invention are therapeutic compositions of matter useful for ameliorating cancer diseases in mammals which contain the acylation products of the present invention as the active ingredients thereof.

This aspect of the invention includes the pharmaceutical compositions and the method of inducing the regression and/or palliation of leukemia and related cancers in mammals when administered in amounts ranging from 0.075 mg to 300 mg per square meter of mammalian body surface area per day. The interrelationship of dosages for animals of various sizes and species and humans (based on $mg/m^2$ of surface area) is described by Freireich, E.J., et al., Quantitative Comparison of Toxicity of Anticancer Agents in Mouse, Rat, Hamster, Dog, Monkey, and Man, Cancer Chemother. Rep., 50, No. 4, 219-244, May 1966. A preferred dosage regimen for optimum results would be from 3.0 $mg/m^2$/day to 150 $mg/m^2$/day. Such dosage units are employed that a total of from 0.5 mg to 525 mg of the active compound for a subject of about 70 kg of body weight are administered in a 24 hour period. This dosage regimen may be adjusted to provide the optimum therapeutic response. For example, several divided doses may be administered daily or the dose may be proportionally reduced as indicated by the exigencies of the therapeutic situation. The active compound may be administered by the intravenous, intramuscular, or subcutaneous routes.

The active compounds may be administered parenterally or intraperitoneally. Solutions or dispersions of the active compound can be prepared in water suitably mixed with a surfactant such as hydroxypropylcellulose. Dispersions can also be prepared in glycerol, liquid polyethylene glycols, and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage, and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol), suitable mixtures thereof, and vegetable oils. The proper fluidity can be maintained, for example, by the use of a coating such as lecithin, by the maintenance of the required particle size in the case of dispersions and by the use of surfactants. The prevention of the action of microorganisms can be brought about by various antibacterial and antifungal agents, for example, parabens, chlorobutanol, phenol, sorbic acid, and thimerosal. In many cases it will be preferable to include isotonic agents, for example sugars or sodium chloride. Prolonged absorption of the injectable compositions can be brought about by the use in the compositions of agents delaying absorption, for example aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from a

previously sterile-filtered solution thereof.

As used herein, "pharmaceutically acceptabale carrier" includes any and all solvents, dispersion media, coatings, antibacterial and antifungal agents, isotonic and absorption delaying agents. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically-acceptable carrier in dosage unit form as hereinbefore disclosed. A unit dosage form can, for example, contain the principal active compound in amounts ranging from 0.1 to 500 mg, with from 10 to 500 mg being preferred. Expressed in proportions, the active compound is generally present in from 0.1 to 100 mg/ml of carrier. In the case of compositions containing supplementary active ingredients, the dosages are determined by reference to the usual dose and manner of administration of the said ingredients.

Regression and palliation of cancers are attained, for example, using intraperitoneal administration. A single intravenous dosage or repeated daily dosages can be administrated. Daily dosages up to about 5 to 10 days are often sufficient. It is also possible to dispense one daily dosage or one dose on alternate or less frequent days. As can be seen from the dosage regimens, the amount of principal active ingredient administered is a sufficient amount to aid regression and palliation of the leukemia, in the absence of excessive deleterious side effects of a cytotoxic natue to the hosts harboring the cancer. As used herein, cancer disease means blood malignancies such as leukemia, as well as other solid and non-solid malignancies such as the melanocarcinomas, lung carcinomas, and mammary tumors. By regression and palliation is meant arresting or retarding the growth of the tumor or other manifestation of the disease compared to the course of the disease in the absence of treatment.

The clinical use of some anti-tumor agents, particularly anthracene derivatives, has been reported to be accompanied by a painful phlebitis in some patients near the site of injection. This phlebitis appears to be associated with the precipitation of the free base of the agent within the blood vessel due to basification of its acid addition salts e.g., the hydrochloride salt, by the blood.

It has now been discovered that the di- and most notably, the monophosphoramidic acid derivatives of the antitumor compound, bisantrene, provided by this invention exhibit excellent clinical efficacy without the attendant phlebitis - precipitation problems seen in the prior art. Both of these derivatives exist as soluble anionic salts at physiological pH, i.e., 7.4. This enhanced solubility apparently reduces if not eliminates altogether any phlebitis reaction near the site of injection. As will be described in the following section, no precipitation of the monophosphoramidic acid compound is detected in a rat tail-vein model.

In the rat, the diphosphoramidic compound was found to be a pro-drug for bisantrene. The diphosphoramidic compound hydrolyzes rapidly to an intermediate form, the monophosphoramidic acid, and this, in turn, slowly hydrolyzes farther to bisantrene after distribution of the drug throughout the animal. These in vivo hydrolyses are apparently enzymatic because the diphosphoramidic compound has been shown to be much more stable in water. Furthermore, the stability in water is more than adequate for an efficient formulation via lyophilization.

This invention will be described in greater detail in conjunction with the following specific examples.

Example 1 (Reference)

Bis(2-imidazolin-2-ylhydrazone) of 9,10-anthracenedicarboxaldehyde

To a solution of 60.0 g of bis(2-imidazolin-2-ylhydrazone) of 9,10-anthracenedicarboxaldehyde dihydrochloride (prepared as described in U.S. Patent 4,258,181) in 1400 ml of water was added a solution of 27.0 g of sodium carbonate in 400 ml of water, with vigorous swirling. The resulting suspension was allowed to stand 5 hours, then the solid was collected in a three-liter, coarse porosity sintered glass funnel

and washed with three 1.2 liter portions of very dilute aqueous ammonia, at a concentration of 2.0 ml of concentrated ammonia per liter. The ammonia solution enabled satisfactorily rapid filtration by lowering surface tension and preventing peptiation of the solid. The last wash was chloride-free and gave 47.2 g of the desired product as a light orange solid, mp 307-308 °C.

Example 2

[9,10-Anthracenediylbis[methylidyne-1-hydrazinyl-2-ylidene(4,5-dihydro-1H-imidazole-2,1-diyl)]]-bisphosphonic acid, tetraethyl ester

To a stirred suspension of 7.969 g of dried bis-(2-imidazolin-2-ylhydrazone) of 9,10-anthracenedicarboxaldehyde in 400 ml of dried dichloromethane under argon was added with stirring via hypodermic syringes and a rubber septum, first 8.137 g of N,O-bis(trimethylsilyl)acetamide, then 6.902 g of diethyl chlorophosphate. The solid all dissolved after stirring for about 3 hours. The solution was filtered through 200 g of dry-packed, air-equilibrated neutral alumina, (ICN, "for dry-column chromatography") in a 3.8cm x 18cm column. The colored part of the eluate was collected (cut 1) and the column was eluted with an additional 5 x 200 ml of dichloromethane to obtain cuts 2-6. Cuts 1-4 were combined and concentrated to 40 ml, then 100 ml of toluene was gradually added to the boiling mixture, with swirling, as a crystalline solid separated and the volume boiled down to 100 ml with bp 100 °C. The solid which crystallized was washed with toluene, then with methanol to give 4.36 g of the desired product as orange needles, mp 217 °C.

Example 3

[9,10-Anthracenediylbis[methylidyne-1-hydrazinyl-2-ylidene(4,5-dihydro-1H-imidazole-2,1-diyl)]]-bisphosphonic acid, tetraphenyl ester

To a stirred suspension of 1.99 g of dried bis-(2-imidazolin-2-ylhydrazone) of 9,10-anthracenedicarboxaldehyde in 100 ml of dried dichloromethane were added, under argon as described in Example 2, 2.03 g of N,0-bis(trimethylsilyl)acetamide "BSA" and 2.68 g of diphenyl phosphorochloridate. After one hour of stirring all of the solid had dissolved. Stirring was continued for 2 hours longer. The reaction solution was poured into a 3.8cm x 18cm dry column of 200 g of air-equilibrated alumina. The column was developed with dichloromethane and the first 100 ml of colorless eluate was discarded, then as the first yellow band neared the bottom, elution cuts of 100 ml each were collected and evaporated. The residue from the first cut, 1.74 g, was dissolved in about 13 ml of dichloromethane, then 40 ml of toluene was added and the solution was heated to boil off the dichloromethane, reduce the volume to about 25 ml and crystallize a solid. The solid was collected by filtration and washed with toluene, then with ether to give 1.65 g of the desired product as an orange solid, mp 214-215 °C.

Example 4

[9,10-Anthracenediylbis[methylidyne-1-hydrazinyl-2-ylidene(4,5-dihydro-1H-imidazole-2,1-diyl)]]-bisphosphonic acid

To a stirred orange solution of 8.74 g of the tetraethyl ester of [9,10-anthracenediylbis[methylidyne-1-hydrazinyl-2-ylidene(4,5-dihydro-1H-imidazole-2,1-diyl)]]-bis phosphonic acid in 150 ml of dried dichloromethane under argon was added 13.0 g of iodotrimethylsilane via a glass hypodermic syringe and rubber septum. There was a slight exothermic reaction to about 40 °C and the solution became yellow. Within 5 minutes it was orange again. After 30 minutes the solution was evaporated to dryness in vacuo. The glassy residue solidified when suspended in 150 ml of acetone containing 5.2 ml of water to hydrolyze the intermediate silyl ester. The suspension was stirred for 16 hours. The solid was collected and washed with acetone to give 8.17 g of yellow solid. This solid was recrystallized by dissolving it in 200 ml of triethylamine, thus forming a soluble phosphoramidic acid salt. The free phosphoramidic acid was precipitated by adding 1.82 ml of 97% formic acid. The solid was collected by filtration and washed with ethanol to give 6.04 g of yellow solid which turned orange when dried, mp 235-238 °C.

Example 5

[9,10-Anthracenediylbis[methylidyne-1-hydrazinyl-2-ylidene (4,5-dihydro-1H-imidazol-2,1-diyl)]] phosphonic acid, diethyl ester

To a 3 liter round bottom flask equipped with a stirring bar was added, under argon, 41.456 g of the bis-(2-imidazolin-2-ylhydrazone) of 9,10-anthracenedicarboxaldehyde, (not specially dried and therefore hydrated), 2 liters of dichloromethane, 42.33 g (51.43 ml of N,0-bis(trimethylsilyl) acetamide via a syringe and 35.90 g (30.07 ml) of diethyl chlorophosphate also via a syringe. After stirring overnight, the cloudy orange mixture was filtered. The filtrate was chromatographed on 1 kg of partially deactivated (air equilibrated) alumina and developed with dichloromethane. Nine 1 liter fractions were taken and partially concentrated. Fractions 1-3 gave the product of Example 2. Fractions 4-7 were combined and further concentrated, giving 13.31 g of solid.

A 13.01 g portion of the above solid in a fritted funnel was washed sparingly with 30, 20 and 10 ml of dichloromethane and then with water, giving 6.50 g of orange solid. This solid was mixed with 200 ml of hot dichloromethane and filtered through 3 g of silica gel, washing with 40 ml of dichloromethane. The filtrate was concentrated to about 30 ml. The resulting solid was collected and washed with a minimum of cold dichloromethane and then with carbon tetrachloride, giving 4.50 g of the desired product as yellow leaflets, mp 195-202 °C. Thin layer chromatography on silica gel vs. chloroform/methanol (9/1), gave a spot with Rf 0.3 as compared with Rf 0.6 for the product of Example 2.

Example 6

[9,10-Anthracenediylbis[methylidyne-1-hydrazinyl-2-ylidene (4,5-dihydro-1H-imidazol-2,1-diyl)]] phosphonic acid hydroiodide

To a solution of 1.07 g of dried [9,10-anthracenediylbis[methylidyne-1-hydrazinyl-2-ylidene (4,5-dihydro-1H-imidazole-2,1-diyl)]]phosphonic acid, diethyl ester and 5.25 g of triphenylphosphine in 90 ml of dried dichloromethane, under argon, was added, via a syringe, 1.0 g (0.71 ml) of iodotrimethylsilane. After 30 minutes, the clear orange solution was evaporated to dryness and then re-evaporated twice from 50 ml portions of dry dichloromethane. The residue was suspended in 50 ml of acetone and 1 ml of water was added, precipitating an orange gum. The gum was pressed thin and allowed to stand overnight in the moist acetone under argon. The gum solidified. It was then pulverized, collected and washed with acetone, giving 1.21 g of the desired product as an orange solid; MS ((+)FAB)507(M+H); NMR (300 $MH_z$, $ME_2SO\text{-}d_6$) $\delta$ 1.23 (t,3,C-$CH_3$), 3.77 (s, $NCH_2CH_2N$), 3.85 (m, $CH_2$of Et), 7.70 (h,4,arom.), 8.44 and 8.49 (m,4,arom.), 8.78 (s,1,NH) , 9.04 (s,1,NH), 9.34 (s,1,CH=N), 9.43 (s,1,CH=N), 12.54 (s,1,C=N'$H^+$).

Example 7

[2-[[[10-[[(4,5-Dihydro-1H-imidazol-2-yl) ethylhydrazono]methyl]-9-anthracenyl]methylene]hydrazino]-4,5-dihydro-1H-imidazol-1-yl]phosphonic acid, hydroiodide

The procedure of Example 6 was followed except that no triphenylphosphine was used to remove by-product ethyl iodide. A solution of the crude, solidified reaction product in 10 ml of methanol was filtered through 1 g of alumina in a 0.6 cm column, washing with 5 ml of methanol. The filtrate was evaporated almost to dryness when the residual syrup began to crystallize. A 20 ml portion of acetone was added, the solid was macerated and then allowed to stand overnight. The solid was collected and washed with acetone, giving 1.057 g of the desired product; MS ((+)FAB), 479(M+H); NMR (300 MHz, $Me_2$ SO-$d_6$) $\delta$ 3.77 (s,8,$NCH_2CH_2N$), 7.66 (m,4,arom.), 8.49 (h,4,arom.), 8.79 (s,2,NH), 8.92 (s,1,NH), 9.34 and 9.36 (d,2, CH=N), 12.58 (d,1,C=N'$H^{\oplus}$).

Example 8

Disodium [9,10-anthracenediylbis [methylidyne-1-hydrazinyl-2-ylidene (4,5-dihydro-1H-imidazole-2,1-diyl)]] bis [phosphate]

To a stirred suspension of 585 mg of the compound prepared according to Example 4, [9,10-anthracenediylbis[methylidyne-1-hydrazinyl-2-ylidene (4,5-dihydro-1H-imidazole-2,1-diyl)]]bisphosphonic

acid in 10 ml of water, monitored by a pH meter, was added dropwise 18.3 ml of 0.1N sodium hydroxide, at a rate such that the pH never exceeded 7.5 and gave a final pH of 7.4. This solution was evaporated at 35 °C over 5 hours, giving 629 mg of the desired product as an amorphous red-orange solid.

The compound prepared according to Example 8 was tested in the rat tail vein model for phlebitis reaction near the site of injection. For comparison purposes, a control placebo and bisantrene were also tested. The compounds were administered intravenously in an amount of 25 mg/kg. Observations were made at 1, 5 and 9 days following the injection.

No evidence of phlebitis was seen in the rat tail vein model that was administered the compound prepared according to Example 8, in contrast to bisantrene which did produce evidence of phlebitis near the site of injection.

**Claims**

**Claims for the following Contracting States : BE, CH, DE, FR, GB, IT, LI, NL, SE**

**1.** A compound of the formula:

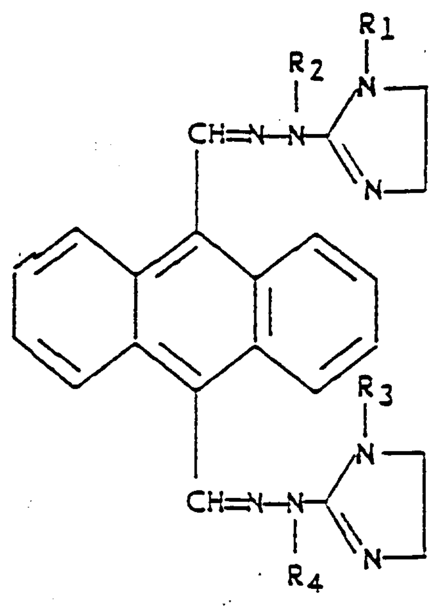

wherein $R_1$ and $R_3$ are the same or different and are hydrogen, alkyl($C_1$-$C_6$),

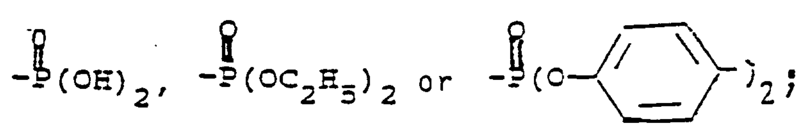

and wherein only one of $R_1$ and $R_3$ may be hydrogen or alkyl($C_1$-$C_6$) ; $R_2$ and $R_4$ are the same or different and are hydrogen, alkyl($C_1$-$C_4$) or

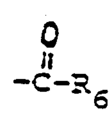

[where $R_6$ is hydrogen, alkyl($C_1$-$C_6$), phenyl, monosubstituted phenyl(wherein the substituent may be in the ortho, meta or para position and is fluoro, nitro, alkyl($C_1$-$C_6$), alkoxy($C_1$-$C_3$) or cyano), pentafluorophenyl, naphthyl, furanyl or -$CH_2OCH_3$]; together with the pharmacologically acceptable salts thereof.

**2.** The compound according to Claim 1,
[9,10-anthracenediylbis[methylidyne-1-hydrazinyl-2-ylidene(4,5-dihydro-1H-imidazole-2,1-diyl)]]-bisphosphonic acid, tetraethyl ester, [9,10-anthracenediylbis[methylidyne-1-hydrazinyl-2-ylidene(4,5-dihydro-1H-imidazole-2,1-diyl)]] bisphosphonic acid, [9,10-anthracenediylbis[methylidyne-1-hydrazinyl-2-ylidene (4,5-dihydro-1H-imidazole-2,1-diyl)]]phosphonic acid, diethyl ester, [9,10-anthracenediylbis-[methylidyne-1-hydrazinyl-2-ylidene (4,5-dihydro-1-H-imidazole-2,1-diyl)]]phosphonic acid or, [2-[[[10-[[-(4,5-di-hydro-1H-imidazol-2-yl)ethylhydrazono]methyl]-9-anthracenyl]methylene]hydrazino]-4,5-dihydro-1H-imidazol-1-yl]-phosphonic acid.

**3.** Use of a compound of Claim 1 in the manufacture of a medicament for treating tumors in warm-blooded animals.

**4.** A pharmaceutical composition in dosage unit form comprising from 0.1 to 500 mg of a compound of Claim 1 in association with a pharmaceutically acceptable carrier.

**5.** A process for preparing a compound of the formula:

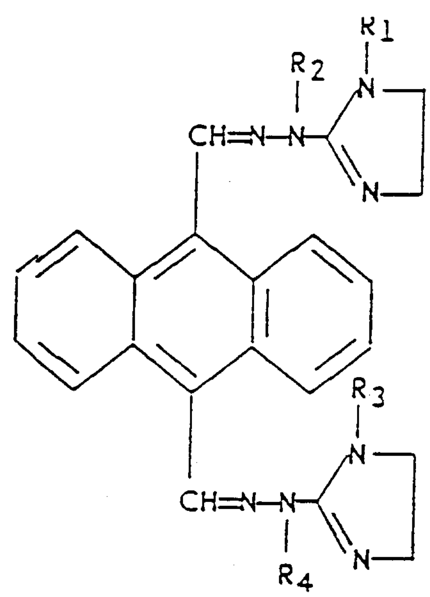

wherein $R_1$ and $R_2$, $R_3$ and $R_4$ are as defined in claim 1 characterized by reacting suspensions of bis-(2-imidazolin-2-ylhydrazones)of9,10-anthracenedicarboxaldehyde in an organic solvent with an acylating agent.

**Claims for the following Contracting States : AT, ES, GR**

**1.** A process for preparing a compound of the formula:

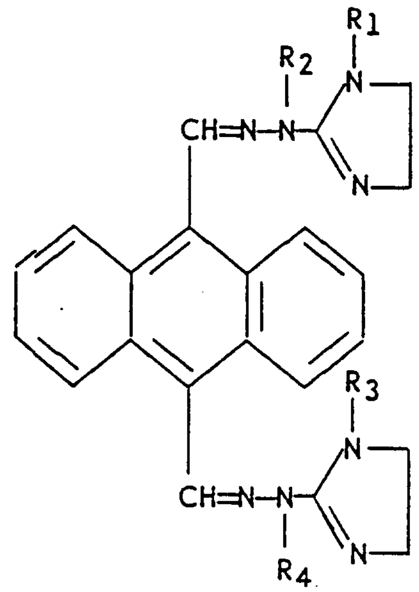

wherein $R_1$ and $R_3$ are the same or different and are hydrogen, alkyl($C_1$-$C_6$),

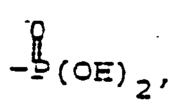 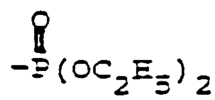 or 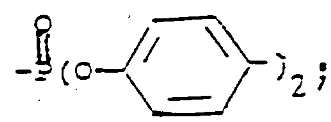

and wherein only one of $R_1$ and $R_3$ may be hydrogen or alkyl($C_1$-$C_6$) ; $R_2$ and $R_4$ are the same or different and are hydrogen, alkyl($C_1$-$C_4$) or

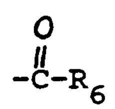

[where $R_6$ is hydrogen, alkyl($C_1$-$C_6$), phenyl, monosubstituted phenyl (wherein the substituent may be in the ortho, meta or para position and is fluoro, nitro, alkyl($C_1$-$C_6$), alkoxy($C_1$-$C_3$) or cyano), pentafluorophenyl, naphthyl, furanyl or -$CH_2OCH_3$]; together with the pharmacologically acceptable salts thereof,
characterized by reacting suspensions of bis(2-imidazolin-2-ylhydrazones)of9,10-anthracenedicarboxaldehyde in an organic solvent with an acylating agent.

**2.** The process according to Claim 1 which produces [9,10-anthracenediylbis[methylidyne-1-hydrazinyl-2-ylidene(4,5-dihydro-1H-imidazole-2,1-diyl)]]-bisphosphonic acid, tetraethyl ester, [9,10-anthracenediylbis[methylidyne-1-hydrazinyl-2-ylidene(4,5-dihydro-1H-imidazole-2,1-diyl)]] bisphosphonic acid, [9,10-anthracenediylbis[methylidyne-1-hydrazinyl-2-ylidene (4,5-dihydro-1H-imidazole-2,1-diyl)]]phosphonic acid, diethyl ester, [9,10-anthracenediylbis-[methylidyne-1-hydrazinyl-2-ylidene (4,5-dihydro-1-H-imidazole-2,1-diyl)]]phosphonic acid or, [2-[[[10-[[-(4,5-di-hydro-1H-imidazol-2-yl)ethylhydrazono]methyl]-9-anthracenyl]methylene]hydrazino]-4,5-dihydro-1H-imidazol-1-yl]-phosphonic acid.

**Patentansprüche**

**Patentansprüche für folgende Vertragsstaaten : BE, CH, DE, FR, GB, IT, LI, NL, SE**

1. Verbindung der Formel:

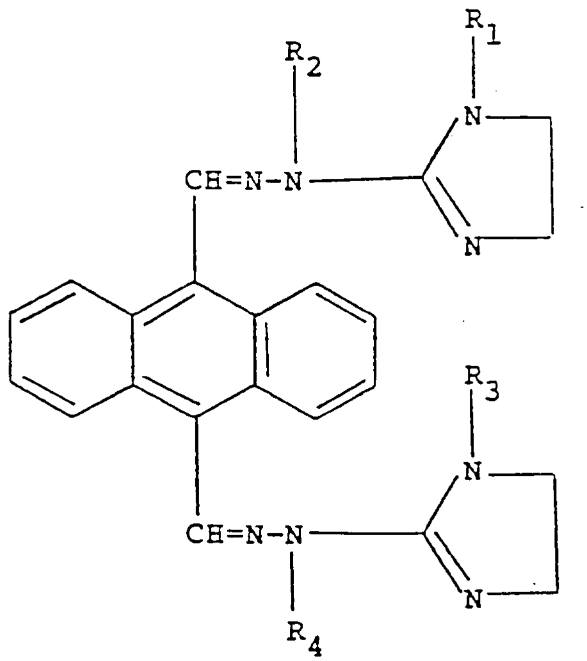

worin $R_1$ und $R_3$ gleich oder verschieden und Wasserstoff, $(C_1\text{-}C_6)$-Alkyl,

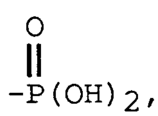, 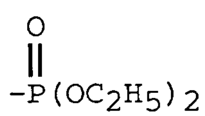 oder 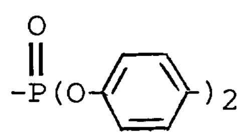

ist, und worin nur eines von $R_1$ und $R_3$ Wasserstoff oder $(C_1\text{-}C_6)$-Alkyl sein kann; $R_2$ und $R_4$ gleich oder verschieden und Wasserstoff, $(C_1\text{-}C_4)$-Alkyl oder

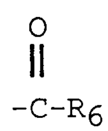

sind, [worin $R_6$ Wasserstoff, $(C_1\text{-}C_6)$-Alkyl, Phenyl, monosubstituiertes Phenyl ist (worin sich der Substituent in der o-, m- oder p-Position befindet und Fluor, Nitro, $(C_1\text{-}C_6)$-Alkyl, $C_1\text{-}C_3$-Alkoxy oder Cyan ist), Pentafluorphenyl, Naphthyl, Furanyl oder $\text{-}CH_2OCH_3$ ist] sowie die pharmakologisch akzeptablen Salze davon.

2. Verbindung nach Anspruch 1, [9,10-Anthracendiylbis[methylidyn-1-hydrazinyl-2-yliden(4,5-dihydro-1H-imidazol-2,1-diyl)]]-bisphosphonsäure-tetraethylester; [9,10-Anthracendiylbis[methylidyn-1-hydrazinyl-2-yliden(4,5-dihydro-1H-imidazol-2,1-diyl)]]bisphosphonsäure; [9,10-Anthracendiylbis[methylidyn-1-hydrazinyl-2-yliden(4,5-dihydro-1H-imidazol-2,1-diyl)]]phosphonsäure-diethylester; [9,10-Anthracendiylbis[methylidyn-1-hydrazinyl-2-yliden(4,5-dihydro-1H-imidazol-2,1-diyl)]]phosphonsäure oder [2-[[[10-[[(4,5-Dihydro-1H-imidazol-2-yl)ethylhydrazono]methyl]-9-anthracenyl]methylen]hydrazino]-4,5-dihydro-1H-imidazol-1-yl]-phosphonsäure.

3. Verwendung einer Verbindung nach Anspruch 1 zur Herstellung eines Medikaments zur Behandlung von Tumoren in warmblütigen Tieren.

4. Pharmazeutische Zusammensetzung in Form einer Dosierungseinheit, umfassend 0,1 bis 500 mg einer Verbindung nach Anspruch 1 in Verbindung mit einem pharmazeutisch akzeptablen Träger.

5. Verfahren zum Herstellen einer Verbindung der Formel:

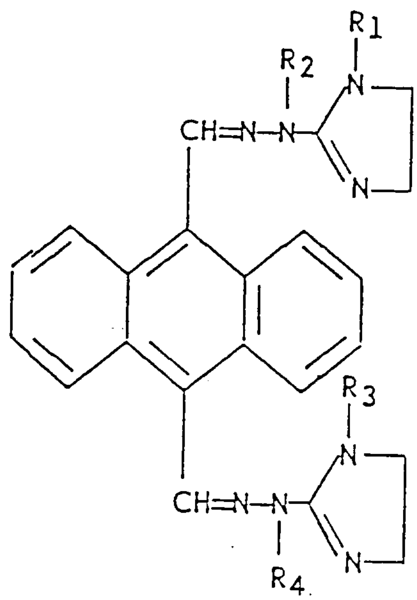

worin $R_1$ und $R_2$, $R_3$ und $R_4$ wie in Anspruch 1 definiert sind, gekennzeichnet durch Umsetzen von Suspensionen von Bis(2-imidazolin-2-ylhydrazonen) von 9,10-Anthracendicarboxaldehyd in einem organischen Lösungsmittel mit einem Acylierungsmittel.

**Patentansprüche für folgende Vertragsstaaten : AT, ES, GR**

1. Verfahren zum Herstellen einer Verbindung der Formel:

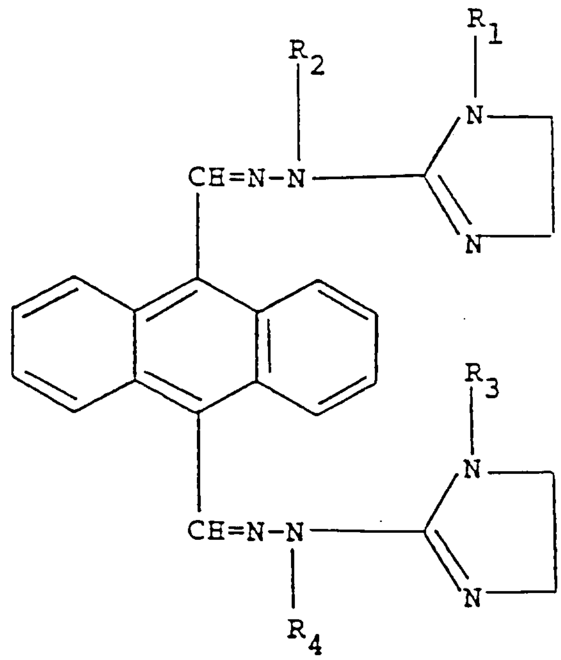

worin $R_1$ und $R_3$ gleich oder verschieden und Wasserstoff, $(C_1\text{-}C_6)$-Alkyl,

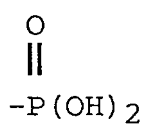, 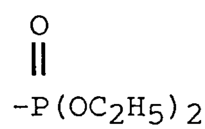 oder 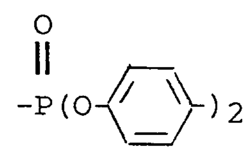

ist, und worin nur eines von $R_1$ und $R_3$ Wasserstoff oder $(C_1\text{-}C_6)$-Alkyl sein kann; $R_2$ und $R_4$ gleich oder verschieden und Wasserstoff, $C_1\text{-}C_4$-Alkyl oder

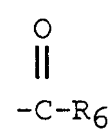

sind, [worin $R_6$ Wasserstoff, $C_1\text{-}C_6$-Alkyl, Phenyl, monosubstituiertes Phenyl ist (worin sich der Substituent in der o-, m- oder p-Position befindet und Fluor, Nitro, $(C_1\text{-}C_6)$-Alkyl, $(C_1\text{-}C_3)$-Alkoxy oder Cyan ist), Pentafluorphenyl, Naphthyl, Furanyl oder $-CH_2OCH_3$ ist] sowie die pharmakologisch akzeptablen Salze davon,
gekennzeichnet durch Umsetzen von Suspensionen von Bis(2-imidazolin-2-ylhydrazonen) von 9,10-Anthracendicarboxaldehyd in einem organischen Lösungsmittel mit einem Acylierungsmittel.

2. Verfahren nach Anspruch 1, das erzeugt [9,10-Anthracendiylbis[methylidyn-1-hydrazinyl-2-yliden(4,5-dihydro-1H-imidazol-2,1-diyl)]]-bisphosphonsäure-tetraethylester; [9,10-Anthracendiylbis[methylidyn-1-hydrazinyl-2-yliden(4,5-dihydro-1H-imidazol-2,1-diyl)]]bisphosphonsäure; [9,10-Anthracendiylbis[methylidyn-1-hydrazinyl-2-yliden(4,5-dihydro-1H-imidazol-2,1-diyl)]]phosphonsäure-diethylester; [9,10-Anthracendiylbis[methylidyn-1-hydrazinyl-2-yliden(4,5-dihydro-1H-imidazol-2,1-diyl)]]phosphonsäure oder [2-[[[10-[[(4,5-Dihydro-1H-imidazol-2-yl)ethylhydrazono]methyl]-9-anthracenyl]methylen]hydrazino]-4,5-dihydro-1H-imidazol-1-y l]-

phosphonsäure.

**Revendications**

**Revendications pour les Etats contractants suivants : BE, CH, DE, FR, GB, IT, LI, NL, SE**

1. Composé de formule :

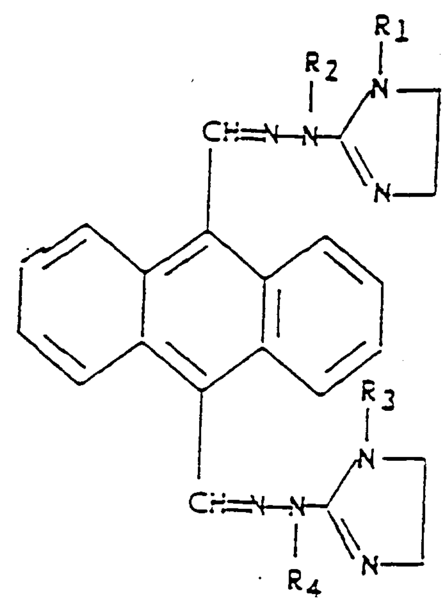

dans laquelle $R_1$ et $R_3$ sont identiques ou différents et sont chacun un atome d'hydrogène, un groupe alkyle en $C_1$-$C_6$,

$$-\overset{O}{\overset{\|}{P}}(OH)_2,\quad -\overset{O}{\overset{\|}{P}}(OC_2H_5)_2,$$ ou 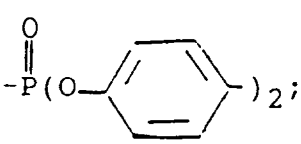

et dans laquelle l'un seulement des radicaux $R_1$ et $R_3$ peut être un atome d'hydrogène ou un groupe alkyle en $C_1$-$C_6$; $R_2$ et $R_4$ sont identiques ou différents et sont chacun un atome d'hydrogène, un groupe alkyle en $C_1$-$C_4$ ou

$$-\overset{O}{\overset{\|}{C}}-R_6$$

[où $R_6$ est un atome d'hydrogène, un groupe alkyle en $C_1$-$C_6$, phényle, phényle mono-substitué (dans lequel le substituant peut être en position ortho, méta ou para et est un groupe fluoro, nitro, alkyle en $C_1$-$C_6$, alcoxy en $C_1$-$C_3$ ou cyano), pentafluorophényle, naphtyle, furanyle ou $-CH_2OCH_3$]; ainsi que les sels pharmacologiquement acceptables de ce composé.

2. Composé selon la revendication 1, qui est l'ester tétraétylique de l'acide [9,10-anthracènediyl-bis-[méthylidyne-1-hydrazinyl-2-ylidène-(4,5-dihydro-1H-imidazole-2,1-diyl)]]bisphosphonique, l'acide [9,10-anthracènediyl-bis[méthylidyne-1-hydrazinyl-2-ylidène-(4,5-dihydro-1H-imidazole-2,1-diyl)] bisphosphonique, l'ester diétylique de l'acide [9,10-anthracènediyl-bis[méthylidyne-1-hydrazinyl-2-ylidène-(4,5-dihydro-1H-imidazole-2,1-diyl)]]phosphonique, l'acide [9,10-anthracènediyl-bis[méthylidyne-1-hydrazinyl-2-ylidène-(4,5-dihydro-1H-imidazole-2,1-diyl)]]phosphonique ou l'acide [2-[[[10-[[(4,5-dihydro-1H-imidazole-2-yl)éthylhydrazono]méthyl]-9-anthracényl]méthylène]hydrazino]-4,5-dihydro-1H-imidazole-1-yl]-

phosphonique.

3. Utilisation d'un composé selon la revendication 1 dans la fabrication d'un médicament pour traiter les tumeurs chez les animaux à sang chaud.

4. Composition pharmaceutique sous forme de doses unitaires, comprenant de 0,1 à 500 mg d'un composé selon la revendication 1, associé à un véhicule pharmaceutiquement acceptable.

5. Procédé de préparation d'un composé de formule :

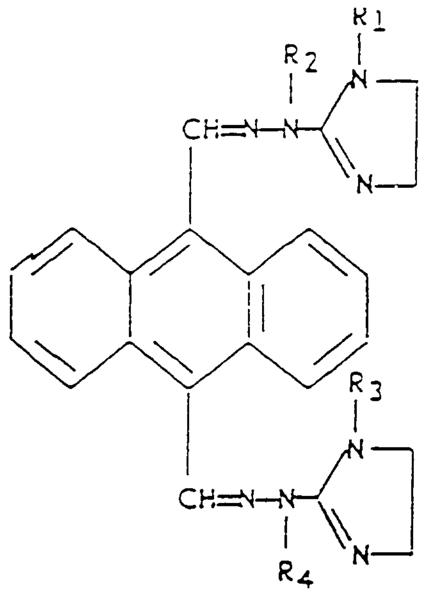

dans laquelle $R_1$, $R_2$, $R_3$ et $R_4$ sont tels que définis dans la revendication 1, caractérisé en ce que l'on fait réagir des suspensions de bis(2-imidazoline-2-ylhydrazones) de 9,10-anthracènedicarboxaldéhyde dans un solvant organique avec un agent d'acylation.

**Revendications pour les Etats contractants suivants : AT, ES, GR**

1. Procédé pour la préparation d'un composé de formule :

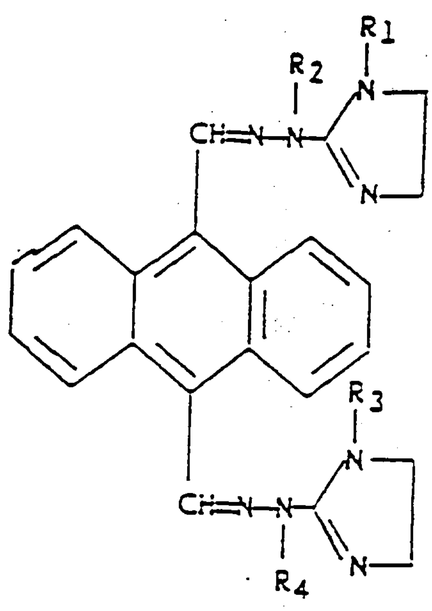

dans laquelle $R_1$ et $R_3$ sont identiques ou différents et sont chacun un atome d'hydrogène, un groupe alkyle en $C_1$-$C_6$,

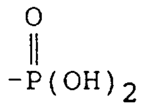, 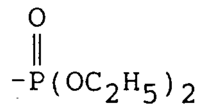, ou 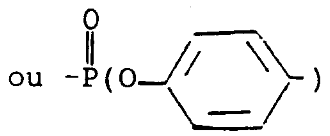;

et dans laquelle l'un seulement des radicaux $R_1$ et $R_3$ peut être un atome d'hydrogène ou un groupe alkyle en $C_1$-$C_6$; $R_2$ et $R_4$ sont identiques ou différents et sont chacun un atome d'hydrogène, un groupe alkyle en $C_1$-$C_4$ ou

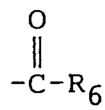

[où $R_6$ est un atome d'hydrogène, un groupe alkyle en $C_1$-$C_6$, phényle, phényle mono-substitué (dans lequel le substituant peut être en position ortho, méta ou para et est un groupe fluoro, nitro, alkyle en $C_1$-$C_6$, alcoxy en $C_1$-$C_3$ ou cyano), pentafluorophényle, naphtyle, furanyle ou $-CH_2OCH_3$]; ainsi que les sels pharmacologiquement acceptables de ce composé,
caractérisé en ce que l'on fait réagir des suspensions de bis(2-imidazoline-2-ylhydrazones) de 9,10-anthracènedicarboxaldéhyle dans un solvant organique avec un agent d'acylation.

2. Procédé selon la revendication 1 pour la préparation de l'ester tétraétylique de l'acide [9,10-anthracènediyl-bis[méthylidyne-1-hydrazinyl-2-ylidène-(4,5-dihydro-1H-imidazole-2,1-diyl)]]bisphosphonique, l'acide [9,10-anthracènediyl-bis[méthylidyne-1-hydrazinyl-2-ylidène-(4,5-dihydro-1H-imidazole-2,1-diyl)]-bisphosphonique, l'ester diétylique de l'acide [9,10-anthracènediyl-bis[méthylidyne-1-hydrazinyl-2-ylidène-(4,5-dihydro-1H-imidazole-2,1-diyl)]]phosphonique, l'acide [9,10-anthracènediyl-bis[méthylidyne-1-hydrazinyl-2-ylidène-(4,5-dihydro-1H-imidazole-2,1-diyl)]]phosphonique ou l'acide [2-[[[10-[[(4,5-dihydro-1H-imidazole-2-yl)éthylhydrazono]méthyl]-9-anthracényl]méthylène]hydrazino]-4,5-dihydro-1H-imidazole-1-yl]phosphonique.